Europäisches Patentamt

European Patent Office

Office européen des brevets

(19)

(11) Veröffentlichungsnummer: **0 160 807**

**A2**

(12) **EUROPÄISCHE PATENTANMELDUNG**

(21) Anmeldenummer: 85102672.4

(22) Anmeldetag: 08.03.85

(51) Int. Cl.⁴: **A 61 M 5/14**

(30) Priorität: 10.05.84 DE 3417257

(43) Veröffentlichungstag der Anmeldung:
13.11.85 Patentblatt 85/46

(84) Benannte Vertragsstaaten:
AT BE CH DE FR GB IT LI LU NL SE

(71) Anmelder: INTERMEDICAT GMBH
Gerliswilstrasse 74
CH-6020 Emmenbrücke(CH)

(72) Erfinder: Lesemann, Egon
Lindenbergstrasse 38
D-3508 Melsungen(DE)

(74) Vertreter: von Kreisler, Alek, Dipl.-Chem. et al,
Deichmannhaus am Hauptbahnhof
D-5000 Köln 1(DE)

(54) **Spülvorrichtung für einen Katheter.**

(57) In dem Gehäuse (10) ist ein zusammendrückbarer Schlauch (18) angeordnet, dessen Enden (18a) und (18b) auf Rohrstücken (22) und (23) sitzen. Zwischen den Rohrstücken (22) und (23) befindet sich ein Kapillarrohr (20) mit einem engen Kapillarkanal (21). Indem ein Druckstück (13) in Querrichtung gegen den Schlauch (18) gedrückt wird, wird dieser flachgedrückt, so daß zwei seitliche Umgehungskanäle entstehen, die um das Kapillarrohr (20) herumführen. Auf diese Weise kann neben dem normaler weise gedrosselten Durchfluß vorübergehend ein stärkerer Durchfluß erreicht werden, indem das Druckstück (13) eingedrückt wird.

FIG.1

## Spülvorrichtung für einen Katheter

Die Erfindung betrifft eine Spülvorrichtung nach dem Oberbegriff des Patentanspruchs 1.

Bei der Katheterisierung von Patienten besteht die Gefahr, daß an dem im Patienten liegenden Katheterende durch Koagulation des Blutes Verstopfungen auftreten, so daß der Durchgang durch den Katheter behindert wird. Die Ursache für solche Verstopfungen kann darin bestehen, daß durch einen vorübergehenden Unterdruck im Katheter Blut oder andere Körperflüssigkeiten in das Katheterende eingesaugt werden. Die Gefahr der Blutgerinnung und des Zusetzens besteht insbesondere bei englumigen Kathetern.

Zum Freihalten des Katheters und zur Verhinderung von Blutgerinnungen am Katheterende ist es bekannt, den Katheter an eine Spülvorrichtung anzuschließen, die eine stetige geringe Flüssigkeitsströmung durch den

Katheter zum Patienten aufrechterhäl⁺ (DE-PS 21 46 588). Die bekannte Vorrichtung weist ein Gehäuse auf, in dem sich ein die Flüssigkeitsströmung drosselndes und dosierendes Kapillarrohr befindet, durch das ständig eine geringe Flüssigkeitsmenge hindurchfließt. Das Gehäuse weist einen Nebenstromweg auf, der durch ein Ventil, das einen elastischen Ventilkörper enthält, verschlossen ist. Dieses Ventil kann durch Ziehen an einem Strang von Hand geöffnet werden. Der Umgehungskanal hat einen wesentlich größeren Durchmesser als das Kapillarrohr, so daß bei geöffnetem Ventil eine erheblich größere Flüssigkeitsmenge pro Zeiteinheit zum Katheter gebracht wird. Die bekannte Vorrichtung benötigt ein relativ großes Gehäuse, in dem zwei Flüssigkeitswege nebeneinander angeordnet sind. Ihre Konstruktion ist relativ aufwendig und ihre Herstellung ist teuer. Derartige Spülvorrichtungen stellen Wegwerfartikel dar, die möglichst kostengünstig hergestellt werden sollten. Ein weiterer Nachteil der bekannten Spülvorrichtung besteht darin, daß zum Öffnen des Umgehungskanals mit zwei Fingern an dem Strang gezogen werden muß, während das Gehäuse mit der anderen Hand festgehalten wird. Zum Öffnen und Geöffnethalten des Umgehungskanals werden also beide Hände benötigt.

Bekannt ist weiterhin eine Spülvorrichtung (DE-PS 32 00 724), bei der das Kapillarrohr in einem drehbaren Ventilkörper des Gehäuses untergebracht ist. In der einen Drehstellung des Ventilkörpers sind Auslaß und Einlaß des Gehäuses über den Kapillarkanal des Kapillarrohrs miteinander verbunden und in einer anderen Drehstellung des Ventilkörpers sind sie über den Umgehungskanal miteinander verbunden. Auch hier benötigt man zum Verstellen des Ventilkörpers beide Hände. Es hat sich herausgestellt, daß es erwünscht ist, nur während einer begrenzten Zeitspanne die Schnellspülung einzuschalten, um anschließend wieder selbsttätig zur Langsamspülung zurückkehren zu können.

Der Erfindung liegt die Aufgabe zugrunde, eine Spülvorrichtung der im Oberbegriff des Patentanspruchs 1 angegebenen Art zu schaffen, die einfach und kostengünstig herstellbar und mit einer Hand leicht zu handhaben ist.

Die Lösung dieser Aufgabe erfolgt erfindungsgemäß mit dem in kennzeichnenden Teil des Patentanspruchs 1 angegebenen Merkmalen.

Der Ventilkörper ist ein Schlauch, der im Ruhezustand das Kapillarrohr umschließt, so daß die durch den Schlauch hindurchfließende Flüssigkeit sämtlich durch den Kapillarkanal des Kapillarrohres hindurchfließen muß. Wird jedoch ein Druck auf das Druckstück ausgeübt, dann wird der Schlauch im Bereich des Kapillarrohres flachgedrückt, wobei er an zwei entgegengesetzten Seiten von der Umfangsfläche des Kapillarrohres abhebt und hier zusätzliche Durchgänge frei macht. Die Flüssigkeit kann nun zusätzlich seitlich an dem Kapillarrohr entlangströmen. Bei einer derartigen Spülvorrichtung ist das Kapillarrohr Bestandteil des Ventils, das als Schlauchventil ausgebildet ist. Das Ventil erfordert somit nahezu keinen zusätzlichen Platz im Gehäuse. Lediglich der Hohlraum, durch den der Schlauch hindurchführt muß im Bereich des Druckstückes ein Übermaß haben, so daß der Schlauch seitlich in den Hohlraum hinaus ausweichen kann, wenn auf das Druckstück ein Druck ausgeübt wird. Die Spülvorrichtung kann somit kleinformatig und mit geringem Aufwand an Material hergestellt werden. Ein besonderer Vorteil besteht darin, daß mit einer einzigen Hand von normaler (gedrosselter) Spülung auf Schnellspülung umgeschaltet werden kann, weil hierzu lediglich mit dem Daumen gegen das Druckstück gedrückt werden muß, während das Gehäuse mit derselben Hand abgestützt wird.

Vorzugsweise ist die Erstreckung der auf den Schlauch einwirkenden Fläche des Druckstücks in Längsrichtung des Kapillarrohres größer als die Länge des Kapillarrohres. Dadurch wird sichergestellt, daß der Schlauch sich gerade auch an den Enden des Kapillarrohres von diesem entfernt, um den Umgehungskanal freizugeben.

Der Hohlraum kann an der dem Druckstück abgewandten Seite des Gehäuseumfangs durch ein Bodenstück abgeschlossen sein, dessen Innenseite die Form einer Wanne hat, deren Weite größer ist als der Durchmesser eines die Schlauchenden aufnehmenden Kanals des Gehäuses. Es ist somit nicht erforderlich, daß der Kanal über seine gesamte Länge eine Weite hat, die das seitliche Ausweichen des radial gepressten Druckstücks ermöglicht. Der Schlauch wird vielmehr an den Enden von dem Kanal eng umschlossen und somit an Verformungen gehindert. Lediglich im Mittelabschnitt des Schlauchs ist der Hohlraum durch das Bodenstück abgeschlossen, das in eine Öffnung des Gehäuses eingesetzt ist und das so gestaltet ist, daß es den Schlauch im Ruhezustand nicht eng umschließt.

Bei einer bevorzugten Ausführungsform der Erfindung sitzen die Schlauchenden abdichtend auf hohlen Rohrstücken, die von entgegengesetzten Seiten her in einen Kanal des Gehäuses hineinragen. Jedes der Rohrstücke kann ein Anschlußelement für eine Schlauchleitung tragen. Die Abdichtung erfolgt zwischen den Schlauchenden und den Rohrstücken, so daß das Gehäuse selbst keine besondere Abdichtung erfordert. Das Gehäuse hat nur die Funktion des Zusammenhaltens und Abstützens der Teile, muß aber selbst nicht flüssigkeitsdicht sein, da keines der Gehäuseteile mit der Flüssigkeit in Berührung kommt.

Die Rohrstücke können sich bis zu dem Kapillarrohr erstrecken. Wichtig ist jedoch, daß zwischen jedem Rohrstück und der Wand des Kapillarrohrs ein radialer Durchlaß vorgesehen ist. Durch diesen Durchlaß kann bei deformiertem Schlauch Flüssigkeit in die zu beiden Seiten des Kapillarrohrs freigewordenen Umgehungsleitungen strömen.

Im folgenden werden unter Bezugnahme auf die Zeichnungen zwei Ausführungsbeispiele der Erfindung näher erläutert.

Es zeigen:

Fig. 1     einen Längsschnitt durch eine erste Ausführungsform der Spülvorrichtung,

Fig. 2     einen Schnitt entlang der Linie II-II von Fig. 1,

Fig. 3     die gleiche Darstellung wie Fig. 2, jedoch bei geöffneten Umgehungswegen,

Fig. 4     einen Längsschnitt durch eine zweite Ausführungsform der Spülvorrichtung und

Fig. 5     einen Schnitt entlang der Linie V-V von Fig. 4.

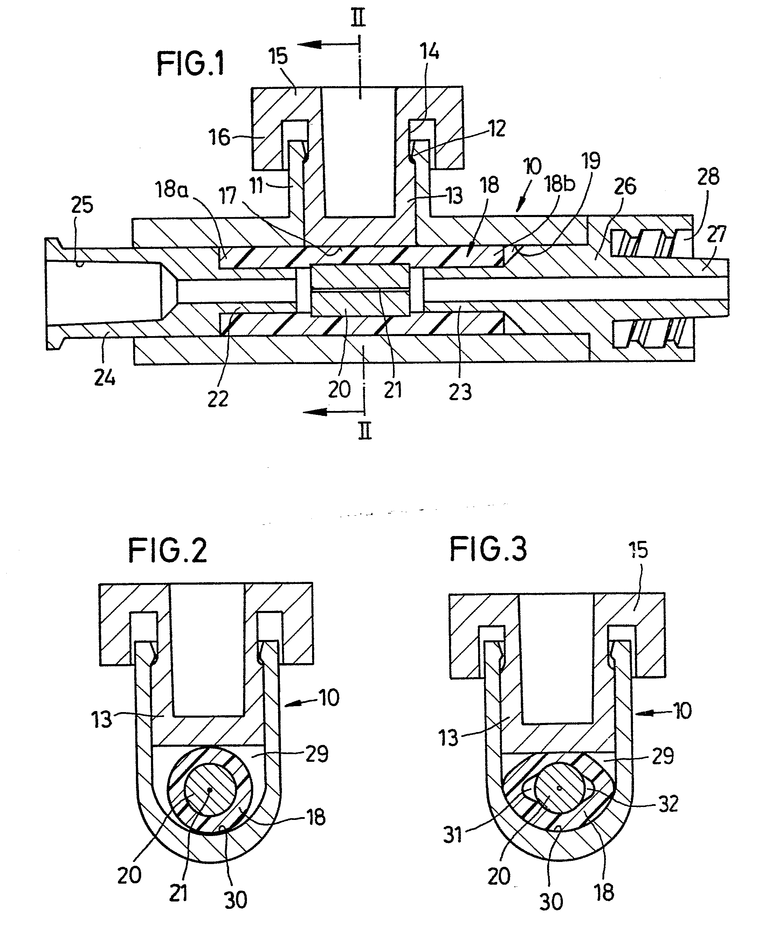

Die Spülvorrichtung nach den Fign. 1 und 2 weist ein rohrförmiges langgestrecktes Gehäuse 10 aus Kunststoff auf, von dem ein Rohrstutzen 11 seitlich abgeht. Am Ende des Rohrstutzens 11 befindet sich ein Innenwulst 12 zum Zurückhalten des Druckstücks 13, das in dem Rohrstutzen 11 verschiebbar ist. Der Innenwulst 12 ragt in eine ringförmige Ausnehmung 14 des Druckstücks 13 hinein. Der Verschiebeweg des Druckstückes 13 wird durch die beiden Enden der Ausnehmung 14 begrenzt. Das Druckstück 13 weist an seinem Ende einen radialen Flansch 15 auf, der einen zurückspringenden Kragen 16 trägt, welcher die Wand des Rohrstutzens 11 mit Abstand übergreift.

Die dem Gehäuseinnern zugewandte Druckfläche 17 des Druckstücks 13 stößt gegen den Schlauch 18, der in dem zylindrischen Kanal 19 des Gehäuses angeordnet ist. Der Außendurchmesser des Schlauchs 18 entspricht dem Innendurchmesser des Kanals 19. Im Mittelbereich des Schlauchs 18 - in Längsrichtung - befindet sich das Kapillarrohr 20, das im vorliegenden Fall aus Glas besteht und einen längslaufenden engen Kapillarkanal 21 aufweist, dessen Durchmesser nur einige hundertstel Millimeter beträgt. Die in Längsrichtung über das Kapillarrohr 20 hinausragenden Enden 18a und 18b des Schlauchs 18 sind jeweils auf ein Rohrstück 22 bzw. 23 aufgezogen. Das Rohrstück 20 ist einem weiblichen Anschlußstück 24 einstückig angeformt, das einen Innenkonus 25 zum Einstecken des Außenkonus eines entsprechenden Gegenstücks aufweist und aus dem Gehäuse 10 herausragt. Das Rohrstück 23 ist einem männlichen Anschlußstück 26 angeformt, das aus dem entgegengesetzten Ende des Kanales 19 aus dem Gehäuse 10 herausragt und einen Anschlußstutzen 27 mit Außenkonus aufweist, der von einer Gewindemuffe 28 umgeben ist. Die Anschlußstücke 24 und 27 sind Bestandteile des bekannten Luer-Systems zum Verbinden medizinischer Schlauchleitungen. Wie aus Fig. 1 zu ersehen ist, enden die Rohrstücke 22 und 23 jeweils in axialem Abstand von dem Kapillarrohr 20. Der Durchmesser des Kapillarrohres 20 ist etwas größer als der Innendurchmesser des Schlauches 18, so daß dieser geringfügig aufgeweitet wird. Die Schlauchenden 18a und 18b sitzen abdichtend auf den Rohrstücken 22 und 23. Wie ferner aus Fig. 1 hervorgeht, ist die Erstreckung des Druckstücks 13 in Längsrichtung des Kapillarrohres 20 größer als die Länge des Kapillarrohres 20, so daß das Druckstück das Kapillarrohr in Längsrichtung an beiden Enden überragt.

Wie aus den Fign. 2 und 3 zu ersehen ist, ist im Gehäuse 10 um das Kapillarrohr 20 herum ein Hohlraum 29 vorgesehen, dessen Weite quer zur Bewegungsrichtung des Druckstücks 13 größer ist als der Außendurchmesser des Schlauchs 18. Der Schlauch 18 liegt mit dem dem Druckstück 13 abgewandten Teil seines Umfangs an der Bodenfläche 30 des Hohlraums 29 an. Die Bodenfläche 30 ist muldenförmig ausgebildet und ihr Durchmesser ist größer als der Außendurchmesser des Schlauchs 18.

Der Schlauch 18 besteht aus einem Elastomermaterial, z.B. aus Silikon-Kautschuk. Wenn auf den Flansch 15 des Druckstücks 13 ein Druck ausgeübt wird, wird der Schlauch 18 um das Kapillarrohr 20 herum flachgedrückt, wobei er sich passend an die Bodenfläche 30 anschmiegt und hierbei beidseitig des Kapillarrohres 20 aufgeweitet wird und von dem Kapillarrohr abhebt. Dadurch bilden sich zwei Umgehungskanäle 31,32 zwischen dem Schlauch 18 und dem Kapillarrohr 20. Diese Umgehungskanäle 31 und 32 überbrücken den Kapillarkanal 21, so daß bei eingedrücktem Druckstück 13 eine erheblich größere Durchflußmenge an dem Kapillarrohr 20 vorbeiströmt als durch dessen Kapillarkanal 21 hindurchfließt. Dies ist der Zustand des Schnellspülens (Fig. 3).

In Fig. 2 ist dagegen das Normalspülen mit geringer Flüssigkeitsmenge dargestellt, wobei der Schlauch 18 das Kapillarrohr 20 an dessem Umfang eng umschließt.

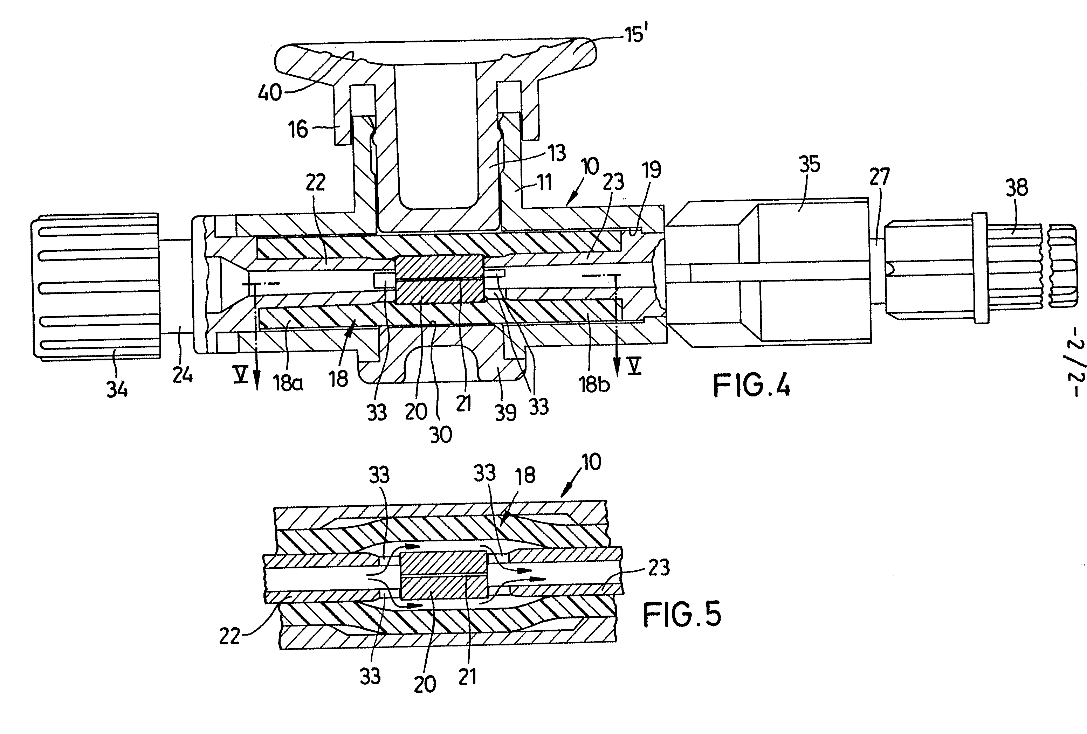

Das Ausführungsbeispiel der Fign. 4 und 5 gleicht weitgehend dem ersten Ausführungsbeispiel, so daß die nachfolgende Beschreibung sich auf die Unterschiede beschränkt.

Die Rohrstücke 22 und 23 erstrecken sich bis zu den Stirnseiten des Kapillarrohres 20. Um den Flüssigkeitsdurchfluß zu den Umgehungskanälen bei flachgedrücktem Schlauch 18 zu ermöglichen, sind an den einander zugewandten Enden der Rohrstücke 22 und 23 Durchbrechungen 33 vorgesehen. Die entgegensetzten Enden der Rohrstücke 22 und 23 sind an den Enden des Kanals 19 durch Kleben befestigt.

Auf das weibliche Anschlußstück 24 ist ein Verschlußkonus 34 aufgeschraubt und das gegenüberliegende Anschlußstück 27 trägt eine Lok-Mutter 35, die frei drehbar ist. Auf dieses Anschlußstück ist eine Schutzkappe 38 aufgesteckt.

Der Kanal 19 hat einen kreisförmigen Querschnitt und sein Durchmesser ist etwas größer als der Außendurchmesser des Schlauchs 18. Die Bodenfläche 30 befindet sich an dem Bodenstück 39, das in eine radiale Öffnung des Kanals 19 eingesetzt und dort durch Kleben befestigt ist. Diese radiale Öffnung ist eine Fortsetzung der Öffnung des Rohrstutzens 11, zu dem sie axial ausgerichtet ist. Das Bodenstück 39 ist daher auf der dem Druckstück 13 gegenüberliegenden Seite des Umfangs des Kanals 19 angeordnet und zwischen beiden befindet sich das Kapillarrohr 20.

Anstelle des Flansches 15 des ersten Ausführungsbeispiels ist der Flansch 15' von Fig. 4 mit einer muldenförmigen Druckfläche 40 versehen, gegen die mit dem Daumen gedrückt werden kann, während das Gehäuse 10 von der entgegengesetzten Seite her abgestützt wird. Bei einem Druck gegen das Druckstück 13 verformt sich der Schlauch 18 in derselben Weise wie dies bei dem ersten Ausführungsbeispiel der Fall ist, so daß die Umgehungskanäle 31 und 32, die an dem Kapillarrohr 20 vorbeiführen, freigegeben werden.

0160807

A N S P R Ü C H E

1. Spühlvorrichtung für einen Katheter, mit einem in einen Flüssigkeitsweg einsetzbaren Gehäuse (10), das ein Kapillarrohr (20) zur Drosselung und Dosierung des Flüssigkeitsstromes aufweist, und mit einem elastischen Ventilkörper, der einen Umgehungskanal zum Kapillarrohr (20) verschließt und durch Krafteinwirkung zum Öffnen des Umgehungskanals verformbar ist, d a d u r c h   g e k e n n z e i c h n e t , daß der Ventilkörper ein das Kapillarrohr (20) umschließender Schlauch (18) ist, daß an dem Gehäuse (10) ein durch Fingerdruck radial zu dem Schlauch (18) verschiebbares Druckstück (13) angebracht ist und daß der das Kapillarrohr (20) und den Schlauch (18) enthaltende Hohlraum (29) des Gehäuses (10) quer zur Bewegungsrichtung des Druckstücks (13) eine größere Weite hat als der Schlauch (18), derart, daß der Schlauch (18) in diesem Hohlraum (29) von dem Druckstück (13) flachdrückbar ist und sich dabei abschnittsweise von dem Umfang des Kapillarrohres (20) entfernt.

2. Spühlvorrichtung nach Anspruch 1, dadurch gekennzeichnet, daß die Erstreckung der auf den Schlauch (18) einwirkenden Fläche (17) des Druckstückes (13) in Längsrichtung des Kapillarrohres (20) größer ist als die Länge des Kapillarrohres.

3. Spühlvorrichtung nach Anspruch 1 oder 2, dadurch gekennzeichnet, daß der Hohlraum (29) an der dem Druckstück (13) abgewandten Seite des Gehäuseumfangs durch ein Bodenstück (39) abgeschlossen ist, dessen Innenseite die Form einer Wanne hat, deren Weite größer ist als der Durchmesser eines die Schlauchenden (18a,18b) aufnehmenden Kanals (19) des Gehäuses (10).

4. Spühlvorrichtung nach einem der Ansprüche 1 bis 3, dadurch gekennzeichnet, daß die Schlauchenden (18a,18b) abdichtend auf hohlen Rohrstücken (22,23) sitzen, die von entgegengesetzten Seiten her in einen Kanal (19) des Gehäuses (10) hineinragen.

5. Spühlvorrichtung nach Anspruch 4, dadurch gekennzeichnet, daß jedes der Rohrstücke (22,23) ein Anschlußelement (24,27) für eine Schlauchleitung trägt.

6. Spühlvorrichtung nach Anspruch 4, dadurch gekennzeichnet, daß zwischen jedem Rohrstück (22,23) und der Wand des Kapillarrohrs (20) ein radialer Durchlaß (33) vorgesehen ist.

FIG.1

FIG.2

FIG.3

FIG.4

FIG.5

0160807